# EUROPÄISCHE PATENTSCHRIFT

(11) **EP 2 016 967 B1**
(45) Veröffentlichungstag und Bekanntmachung des Hinweises auf die Patenterteilung: **25.01.2012**
(21) Anmeldenummer: 08156358.7
(22) Anmeldetag: 16.05.2008
(51) Int. Cl.: A61M 16/00, G06F 19/00

(54) **Verfahren zur Übertragung von Überwachungszuständen**
Method for transmitting surveillance statuses
Procédé destiné à la transmission d'états de surveillance

(30) Priorität: 20.07.2007 DE 102007034018
(43) Veröffentlichungstag der Anmeldung: 21.01.2009
(73) Patentinhaber: Dräger Medical GmbH, 23558 Lübeck (DE)
(72) Erfinder: Dr. Schermeier, Olaf, 23560 Lübeck (DE); Heesch, Ralf, 23568 Lübeck (DE); Schmid, Robert, 23562 Lübeck (DE); Lünse, Jennifer, 23568 Lübeck (DE)
(74) Vertreter: UEXKÜLL & STOLBERG

(56) Entgegenhaltungen:
- EP-A- 1 731 188
- DE-A1- 19 833 443
- DE-B3-102006 056 723
- US-A1- 2002 049 371
- US-B1- 6 364 834

## Beschreibung

Die Erfindung betrifft ein Verfahren zur Übertragung von Überwachungszuständen zwischen Atemvorrichtungen.

Moderne Atemvorrichtungen zeichnen sich durch eine zunehmende Komplexität hinsichtlich der Überwachung und Versorgung der Patienten aus. Sie weisen Überwachungseinheiten auf, die verschiedenste Größen aufzeichnen, wie beispielsweise Atemwegsdrücke, Atemgaskonzentrationen oder Sauerstoffsättigungen des Patientenblutes. Die Überwachungseinheiten der Atemvorrichtungen kontrollieren dabei die Messwerte innerhalb der eingestellten Grenzwerte und informieren den Anwender bei Veränderungen. In neueren Atemvorrichtungen sind die Überwachungseinheiten der Atemvorrichtungen mit intelligenten Algorithmen ausgestattet, die automatisch erkennen, ob eine bestimmte Überwachungsgröße überwacht werden soll. Dazu wird beispielsweise detektiert, ob ein dafür erforderlicher Sensor mit dem Patienten verbunden worden ist und entsprechende Messergebnisse liefert. Erst nachdem alle für die Überwachung der Überwachungsgröße notwendigen Kriterien erfüllt sind, erfolgt eine Aktivierung der Überwachungseinheit. Wird beispielsweise ein Patient in einem Rettungswagen an eine Atemvorrichtung angeschlossen, erfolgt somit eine Überwachung der Sauerstoffsättigung des Blutes (SPO₂ - Wert) erst nachdem ein Sensor zur Messung des SPO₂-Wertes am Patienten angeschlossen worden ist und die Überwachungseinheit einen Puls des Patienten detektiert hat. Bei einem Anschluss des Patienten an eine weitere zweite Atemvorrichtung nach einem Transport in einen Operationsraum einer Klinik, wird der SPO₂- Wert erst wieder überwacht, wenn die Überwachungseinheit einen an den Patienten angeschlossen Sensor und einen Puls von diesem Patienten erkannt hat. Hat sich jedoch der Zustand des Patienten beim Wechsel zwischen den Atemvorrichtungen erheblich dass kein Puls von der Überwachungseinheit erkannt werden konnte, erfolgt aufgrund der automatischen Überwachungssteuerung keine Überwachung der SPO₂ Messung. Der Anwender wird hinsichtlich der fehlenden SPO₂ Messung und / oder des verschlechterten Zustandes des Patienten nicht alarmiert. Dieser Zustand kann zur Beeinträchtigung des Patienten führen.

Aus der DE 10 2006 056 723 B3 ist ein medizinisches System mit einer Überwachungsstation für eine zentrale Datenerfassung eines ersten und eines zweiten medizinischen Arbeitsplatzes bekannt. Die medizinischen Arbeitsplätze sind jeweils ausgebildet, physiologische Daten, insbesondere Respirationsdaten eines Patienten zu erfassen. Das medizinische System ist ferner ausgebildet, die physiologischen Daten und Patientendaten in Abhängigkeit eines Markierungssignals einander zuzuordnen und an der Überwachungsstation zur Anzeige zu bringen.

Aus der DE 198 33 443 A1 ist eine Atemvorrichtung bestehend aus einer Messstation zur Verbindung mit verschiedenen ortsfesten Auswertestation bekannt. Die Messstation kann beim Transport eines Patienten von einer ersten Auswertestation abgenommen werden, um danach mit einer weiteren Auswertestation verbunden zu werden. Eine Übertragung von Überwachungszuständen zwischen den verschiedenen Atemvorrichtungen ist nicht erforderlich, da die gesamte Messstation mit dem Patienten zusammen transportiert wird.

Der Erfindung liegt die Aufgabe zugrunde, die bestehenden Nachteile im Stand der Technik der sich in der Präambel von Anspruch 1 wiederspiegelt zu überwinden.

Die Lösung der Aufgabe erhält man mit den im kennzeichnenden Teil von Anspruch 1 angegebenen Verfahrensmerkmalen.

Die Unteransprüche geben vorteilhafte Ausgestaltungen des Verfahrens nach Anspruch 1 an.

Die Erfindung besitzt den wesentlichen Vorteil, dass bei einem Wechsel eines Patienten von einer ersten zu einer zweiten Atemvorrichtung die Überwachungszustände wenigstens eines ersten Überwachungsabschnittes der ersten Atemvorrichtung für die Einstellung der Überwachungseinheit der zweiten Atemvorrichtung berücksichtigt werden und damit die Gefahr sowohl von Fehleinstellungen als auch von Fehlbedienungen an Atemvorrichtungen minimiert und somit die Sicherheit der Patienten signifikant erhöht wird. Ein Überwachungsabschnitt ist ein zeitlicher Abschnitt zur Messung einer zu überwachenden Größe.

In zweckmäßiger Weise wird dabei neben den im Verfahrensschritt a) erfassten Daten auch die im Verfahrensschritt b) zugewiesene Kennung an ein Überwachungsinformationssystem übertragen, wobei die im Verfahrensschritt a) erfassten Daten unter der im Verfahrensschritt b) zugewiesenen Kennung abgelegt werden. Die im Verfahrensschritt b) zugewiesene Kennung kann auf ein auslesbares Speicherelement eines Zubehörteils, vorzugsweise eines Atemschlauchs, von einer Sendeeinheit hinterlegt werden. Der Atemschlauch wird mit der zweiten Atemvorrichtung verbunden, wobei von einer Empfangseinheit der zweiten Atemvorrichtung die auf dem Speicherelement des Atemschlauchs hinterlegte Kennung gelesen werden kann. Die Überwachungszustände des wenigstens ersten Überwachungsabschnittes der ersten Atemvorrichtung können von der zweiten Atemvorrichtung unter dieser Kennung von dem Überwachungsinformationssystem angefordert werden und auf die zweite Atemvorrichtung übertragen werden.

In vorteilhafter Weise sind dabei die erste Atemvorrichtung, die zweite Atemvorrichtung und das Überwachungsinformationssystem über ein Netzwerk miteinander verbunden. Die erste Atemvorrichtung, die zweite Atemvorrichtung und das Überwachungsinformationssystem können auch drahtlos miteinander in Verbindung stehen. Bei der Einstellung der Überwachungseinheit der zweiten Atemvorrichtung entsprechend der übertragenen Überwachungszustände der Überwachungseinheit des Überwachungsabschnittes der ersten Atemvorrichtung werden in vorteilhafter Weise die gegenwärtigen Überwachungsgrößen der zweiten Atemvorrichtung berücksichtigt.

Ein Überwachungszustand kann dabei vorzugsweise eine Aktivierung und /oder eine Deaktivierung wenigstens einer Überwachungsgröße umfassen. Als Überwachungsgrößen werden insbesondere eine SPO₂ - Messung, eine CO₂ - Messung, eine O₂ - Messung und / oder eine Anästhesiemittelkonzentrationsmessung angesehen. Die Aktivierung und / oder Deaktivierung einer Überwachungsgröße der ersten Atemvorrichtung wird somit registriert und bei der Einstellung der Überwachungseinheit der zweiten Atemvorrichtung berücksichtigt.

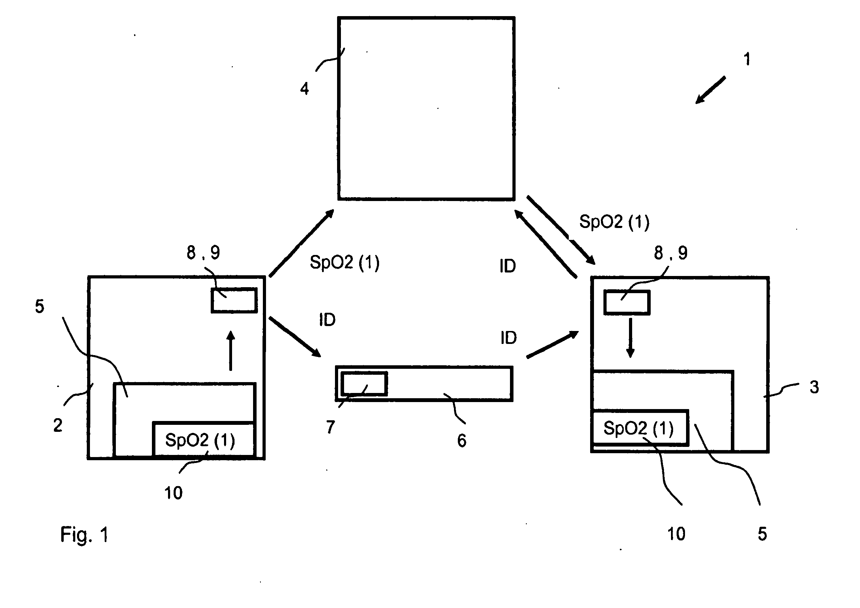

Eine Vorrichtung zur Durchführung des erfindungsgemäßen Verfahrens ist in der Figur 1 gezeigt. Die Erfindung wird im Folgenden an zwei Ausführungsbeispielen eines Beatmungssystems 1 näher erläutert.

Eine Überwachungseinheit 5 erfasst die Überwachungszustände einer ersten Atemvorrichtung 2, die einen in einem Operationsvorbereitungsraum befindlichen Patienten mit Atemgas versorgt. Den Überwachungszuständen eines ersten Überwachungsabschnittes 10 wird eine Kennung zugewiesen. Die Kennung kann beispielsweise den Namen des Patienten, eine Behandlungsnummer oder eine Patientenidentifikationsnummer umfassen. Die Überwachungszustände des ersten Überwachungsabschnittes 10 der ersten Atemvorrichtung 2 umfassen die Messgrößen und Einstellungen einer SPO₂ - Messung und einer Anästhesiegasmessung. Die erste Atemvorrichtung 2 ist über ein Datennetz mit einem Überwachungsinformationssystem 4 verbunden. Wird die Überwachungseinheit 5 der ersten Atemvorrichtung 2 bei Erkennung eines an den Patienten angeschlossenen SPO₂ - Sensors und des Pulses des Patienten aktiviert, so werden die Überwachungszustände via Datennetz an das Überwachungsinformationssystem 4 übertragen. Die Überwachungszustände beinhalten Daten über Messwerte, einer Aktivierung einer Messung und der Zeitdauer der bereits erfolgten Messungen. Die Überwachungszustände werden auf dem Überwachungsinformationssystem 4 unter der zugewiesenen Kennung abgelegt. Es können aber auch in vorteilhafter Weise Überwachungszustände weiterer Überwachungsabschnitte 10 an das Überwachungsinformationssystem 4 übertragen und abgelegt werden. Dafür können zwei Kennungen vergeben werden: eine erste Kennung, die einen zu überwachenden Patienten kennzeichnet und eine zweite Kennung, die den jeweiligen Überwachungsabschnitt 10 der ersten Atemvorrichtung 2 kennzeichnet. Zudem kann eine Kennzeichnung der ersten Atemvorrichtung zusammen mit den Kennungen übertragen werden. Die zugewiesenen Kennungen werden mit einer Sendeeinheit 8 der ersten Atemvorrichtung 2 auf ein auslesbares Speicherelement 7 abgelegt. Das Speicherelement 7 befindet sich vorzugsweise in der Ummantelung eines an die erste Atemvorrichtung 2 angeschlossenen Atemschlauches 6. Das Speicherelement 7 kann aber auch an der Außenfläche eines an der ersten Atemvorrichtung 2 angeschlossenen Atemschlauches 6 vorgesehen sein. Nachdem der Patient in einen Operationsraum transportiert wurde, wird der Atemschlauch 6 von der ersten Atemvorrichtung 2 entfernt und an eine in dem Operationsraum befindliche zweite Atemvorrichtung 3 angeschlossen. Die zweite Atemvorrichtung 3 ist dabei mit einer Empfangseinheit 9 zur Auslesung der in dem Speicherelement 7 des Atemschlauches 6 hinterlegten Daten versehen. Die zweite Atemvorrichtung 3 ist gleichzeitig über ein Netzwerk mit dem Überwachungsinformationssystem 4 verbunden. Nach dem Einlesen der Kennung von dem Speicherelement 7 können von der zweiten Atemvorrichtung 3 die Überwachungszustände des ersten Überwachungsabschnittes 10 der ersten Atemvorrichtung 2 von dem Überwachungsinformationssystem 4 angefordert und in die zweite Atemvorrichtung 3 übertragen werden. Nach der erfolgten Übertragung wird der Überwachungszustand der ersten Atemvorrichtung 2 von der zweiten Atemvorrichtung 3 ausgewertet. Hierzu werden in vorteilhafter Weise die gegenwärtigen Überwachungsgrößen der zweiten Atemvorrichtung 3 bei der Einstellung der Überwachungseinheit 5 der zweiten Atemvorrichtung 3 berücksichtigt. Aus dem Überwachungszustand der ersten Atemvorrichtung 2 ist für die Überwachungseinheit 5 der zweiten Atemvorrichtung 3 erkennbar, dass bereits eine SPO₂ - Messung und eine Atemgasüberwachung an der ersten Atemvorrichtung 2 stattgefunden haben. Erfolgt an der zweiten Atemvorrichtung 3 keine Aktivierung der SPO₂ - Messung in einer vordefinierten Zeit aufgrund des erkannten SPO₂ - Sensors und der Pulse des Patienten, beziehungsweise liefert eine SPO₂ - Messung keine Messwerte, dann wird von der Überwachungseinheit 5 der zweiten Atemvorrichtung 3 ein entsprechendes Alarmsignal ausgelöst. Das Alarmsignal signalisiert dem Anwender, dass entweder der SPO₂ - Sensor nicht an der zweiten Atemvorrichtung 3 und / oder an dem Patienten angeschlossen ist oder dass kein Puls von dem Patienten detektiert wird, beispielsweise aufgrund einer signifikanten Verschlechterung des Zustand des Patienten während des Transportes von dem Vorbereitungsraum in den OP-Raum. Damit ist beispielsweise ein nicht erkannter Sauerstoffmangel im arteriellen Blut oder im Gewebe des Patienten (eine Hypoxie) nur noch unter Nichtbeachtung des entsprechenden Alarmsignals von der zweiten Atemvorrichtung 3 durch den Anwender gegeben. Mit dem erfindungsgemäßen Verfahren können somit wirkungsvoll Sicherheitslücken in der Überwachung eines Patienten geschlossen werden.

Die Überwachungsgrößen eines Überwachungszustandes können dabei eine CO₂ - Messung und / oder eine O₂ - Messung umfassen. Das erfindungsgemäße Verfahren ist jedoch nicht alleinig auf diese genannten Überwachungsgrößen limitiert. Als Überwachungsgrößen eines Überwachungszustandes können beispielsweise auch EKG-, BIS-, EEG-, Blutdruck - Signale, ein Atemvolumen, ein Atemdruck, eine Art der Anästhesiegase und / oder eine Narkose- bzw. Hypnosetiefe vorgesehen sein.

Das zweite Ausführungsbeispiel beschreibt eine Überwachungseinheit 5 eines ersten Atemgerätes 2 eines Beatmungssystems 1, bei dem der Therapieverlauf an einem Patienten modellhaft analysiert wird. Aus den Analyseergebnissen werden die von der Überwachungseinheit 5 zu kontrollierenden Grenzwerte abgeleitet. Derartige Überwachungseinheiten 5 sind bei der so genannten "Awareness Prevention" Überwachung vorzufinden. Der Begriff "Awareness" bezeichnet dabei die Wachheit des Patienten während einer Narkose. Mit einer "Awareness Prevention" Überwachung soll ein Wachzustand während einer Narkose vermieden werden. Die Überwachungseinheit 5 erkennt dabei aus dem Verlauf der inspiratorischen und der exspiratorischen Anästhesiegas-Messwerte, ob die derzeitig aktive Atemvorrichtung die Quelle der Anästhesiegaslieferung für den Patienten bildet. Tritt dieser Fall ein, wird weiterhin modellhaft, basierend auf den exspiratorischen Anästhesiegasmesswerten und den Zeiträumen, in denen diese Werte gemessen worden sind, analysiert, wie stark der Patient durch diese Anästhesiegaslieferung in die Narkose versetzt worden ist. Ist der Patient in einen medizinisch relevanten Narkosezustand versetzt worden, so wird eine bestimmte Alarmgrenze von der Überwachungseinheit 5 aktiviert, die bezogen auf den Narkosezustand des Patienten überwacht wird. Stellt die erste Atemvorrichtung 2 die Narkosegaslieferung aufgrund fehlerhafter Versorgung oder durch den Anwender bedingt ein, kann eine Warnung durch die Überwachungseinheit 5 des Anwenders rechtzeitig vor dem Erwachen des Patienten erfolgen. Die Daten des Überwachungszustandes der "Awareness Prevention" Überwachung werden nach Zuweisung einer entsprechenden Kennung von der ersten Atemvorrichtung 2 einem Überwachungsinformationssystem 4 zur Verfügung gestellt. Die Übertragung der Daten kann ebenso wie im ersten Ausführungsbeispiel leitungsgebunden über ein Netzwerk oder über eine entsprechende Funkverbindung erfolgen. Die Daten werden unter der vergebenen Kennung auf dem Überwachungsinformationssystem 4 abgelegt. Die Kennung wird von einer Sendeeinheit 8 der ersten Atemvorrichtung 2 an ein in einem Atemschlauch 6 vorgesehenes Speicherelement 7 übertragen. Bei einem Wechsel des Patienten an eine zweite Atemvorrichtung 3, beispielsweise bei einer Verlagerung des Patienten von dem Operationsraum auf die Intensivstation, verbleibt der Atemschlauch 6 beim Patienten. Nach dem Anschluss des Patienten an die zweite Atemvorrichtung 3 wird die in dem Speicherelement 7 hinterlegte Kennung durch eine Empfangseinheit 9 der zweiten Atemvorrichtung 3 erfasst. Mittels dieser Kennung können die Daten des Überwachungszustandes der "Awareness Prevention" Überwachung der ersten Atemvorrichtung 2 von dem Überwachungsinformationssystem 4 angefordert und in die zweite Atemvorrichtung 3 übertragen werden. Aus diesen Daten und den gegenwärtigen Daten der zweiten Atemvorrichtung 3 wird eine Einstellung der "Awareness Prevention" Überwachung an der zweiten Atemvorrichtung 3 vorgenommen. Werden hierbei von der zweiten Atemvorrichtung 3 Werte ermittelt, die von den Werten abweichen, die durch die Überwachungseinheit 5 der ersten Atemvorrichtung 2 bestimmt worden sind, erfolgt eine Alarmierung des Anwenders der zweiten Atemvorrichtung 3.

Mit dem erfindungsgemäßen Verfahren lässt sich die Überwachungseinheit 5 einer zweiten Atemvorrichtung 3 optimal für eine sichere Patientenüberwachung einstellen.

### BEZUGSZEICHENLISTE

- 1: ... Beatmungssystem
- 2: ... erste Atemvorrichtung
- 3: ... zweite Atemvorrichtung
- 4: ... Überwachungsinformationssystem
- 5: ... Überwachungseinheit
- 6: ... Atemschlauch
- 7: ... Speicherelement
- 8: ... Sendeeinheit
- 9: ... Empfangseinheit
- 10: ... Überwachungsabschnitt

## Patentansprüche

1. Verfahren zur Übertragung von Überwachungszuständen zwischen Atemvorrichtungen, das die folgenden Verfahrensschritte aufweist:
a) Erfassung und Aufzeichnung von Überwachungszuständen von wenigstens einem Überwachungsabschnitt (10) einer Überwachungseinheit (5) einer ersten Atemvorrichtung (2) und
b) zuweisung wenigstens einer Kennung an die Überwachungszustände des wenigstens einen Überwachungsabschnittes (10) der Überwachungseinheit (5) der ersten Atemvorrichtung,
**gekennzeichnet durch** eine
c) Übertragung der wenigstens einen Kennung und Überwachungszustände des wenigstens einen Überwachungsabschnittes (10) der Überwachungseinheit (5) der ersten Atemvorrichtung (2) an eine zweite Atemvorrichtung (3) und
d) Einstellung der Überwachungseinheit (5) der zweiten Atemvorrichtung (3) entsprechend der übertragenen Überwachungszustände des wenigstens einen Überwachungsabschnittes (10) der Überwachungseinheit (5) der ersten Atemvorrichtung (2), wobei die gegenwärtigen Überwachungszustände der Überwachungseinheit (5) der zweiten Atemvorrichtung (3) derart bei der Einstellung der Überwachungseinheit (5) der zweiten Atemvorrichtung (3) berücksichtigt werden, dass eine Abweichung der gegenwärtigen Überwachungszustände der zweiten Atemvorrichtung (3) von den Überwachungszuständen des wenigstens einen Überwachungsabschnittes (10) der ersten Atemvorrichtung (2) von der zweiten Atemvorrichtung (3) signalisiert wird.

2. Verfahren nach Anspruch 1, **dadurch gekennzeichnet, dass** die Schritte a) bis d) für die Überwachungszustände jedes weiteren Überwachungsabschnittes (10) der Überwachungseinheit (5) der ersten Atemvorrichtung (2) wiederholt werden.

3. Verfahren nach einem der vorhergehenden Ansprüche, **dadurch gekennzeichnet, dass** im Verfahrensabschnitt b) zwei Kennungen an die Überwachungszustände des wenigstens einen Überwachungsabschnittes (10) der Überwachungseinheit (5) der ersten Atemvorrichtung (2) zugewiesen werden, wobei vorzugsweise eine Kennung den Patienten und eine Kennung den wenigstens einen Überwachungsabschnitt (10) kennzeichnet.

4. Verfahren nach einem der vorhergehenden Ansprüche, **dadurch gekennzeichnet, dass** die im Verfahrensabschnitt a) erfassten Überwachungszustände des wenigstens einen Überwachungsabschnittes (10) zusammen mit der im Verfahrensschritt b) zugewiesenen Kennung an ein Überwachungsinformationssystem (4) übertragen werden, wobei die im Verfahrensabschnitt a) erfassten Überwachungszustände des wenigstens einen Überwachungsabschnittes (10) unter der im Verfahrensschritt b) zugewiesenen Kennung auf dem Überwachungsinformationssystem (4) abgelegt werden.

5. Verfahren nach einem der vorhergehenden Ansprüche, **dadurch gekennzeichnet, dass** Daten zur Kennzeichnung der Überwachungszustände des wenigstens einen Überwachungsabschnittes (10) der Überwachungseinheit (5) der ersten Atemvorrichtung (2) auf ein auslesbares Speicherelement (7) hinterlegt werden.

6. Verfahren nach Anspruch 5, **dadurch gekennzeichnet, dass** das Speicherelement (7) in einem zubehörteil vorgesehen ist, wobei das Zubehörteil vorzugsweise als Atemschlauch (6) ausgebildet ist.

7. Verfahren nach Anspruch 6, **dadurch gekennzeichnet, dass** im Verfahrensschritt c) das Zubehörteil mit der zweiten Atemvorrichtung (3) verbunden wird, wobei von einer Empfangseinheit (9) der zweiten Atemvorrichtung (3) die Daten des Speicherelementes (7) des Zubehörteils gelesen werden.

8. Verfahren nach den Ansprüchen 3 und 7, **dadurch gekennzeichnet, dass** die Überwachungszustände des wenigstens einen Überwachungsabschnittes (10) der ersten Atemvorrichtung (2) von der zweiten Atemvorrichtung (3) unter der Kennung von dem Überwachungsinformationssystem (4) angefordert und an die zweite Atemvorrichtung (3) übertragen werden.

9. Verfahren nach einem der vorhergehenden Ansprüche, **dadurch gekennzeichnet, dass** die erste Atemvorrichtung (2), die zweite Atemvorrichtung (3) und das Überwachungsinformationssystem (4) über ein Datennetz miteinander verbunden sind.

10. Verfahren nach einem der vorhergehenden Ansprüche, **dadurch gekennzeichnet, dass** ein Überwachungszustand eine Aktivierung und / oder eine Deaktivierung wenigstens einer Überwachungsgröße umfasst.

11. Verfahren nach Anspruch 10, **dadurch gekennzeichnet, dass** die wenigstens eine Überwachungsgröße eine Sp02 - Messung, eine C02 - Messung, eine 02 - Messung und/oder eine Anästhesiemittelkonzentrationsmessung umfasst.

12. Verfahren nach Anspruch 10, **dadurch gekennzeichnet, dass** im Verfahrensschritt d) die Aktivierung und / oder die Deaktivierung der wenigstens einen Überwachungsgröße berücksichtigt werden.

13. Verfahren nach einem der vorhergehenden Ansprüche, **dadurch gekennzeichnet, dass** im Verfahrensschritt d) wenigstens eine Überwachungsgröße aktiviert wird und vorzugsweise die Aktivierung der wenigstens einen Überwachungsgröße zeitverzögert erfolgt.

## Claims

1. A Method for transmitting monitoring statuses between breathing apparatuses comprising the following method steps:
a) detecting and recording of monitoring statuses of at least one monitoring period (10) of a monitoring unit (5) of a first breathing apparatus (2), and
b) assigning at least one identifier to the monitoring statuses of the at least one monitoring period (10) of the monitoring unit (5) of the first breathing apparatus,
**characterized by** a
c) transmission of the at least one identifier and monitoring statuses of the at least one monitoring period (10) of the monitoring unit (5) of the first breathing apparatus (2) to a second breathing apparatus (3), and
d) adjustment of the monitoring unit (5) of the second breathing apparatus (3) according to the transmitted monitoring statuses of the at least one monitoring period (10) of the monitoring unit (5) of the first breathing apparatus (2), wherein the present monitoring statuses of the monitoring unit (5) of the second breathing apparatus (3) are taken into account in the adjustment of the monitoring unit (5) of the second breathing apparatus (3) in such a manner that a deviation of the present monitoring statuses of the second breathing apparatus (3) from the monitoring statuses of the at least one monitoring period (10) of the first breathing apparatus (2) is signaled by the second breathing apparatus (3).

2. The method according to claim 1, **characterized in that** the steps a) to d) are repeated for the monitoring statuses of each further monitoring period (10) of the monitoring unit (5) of the first breathing apparatus (2).

3. The method according to any of the preceding claims, **characterized in that** in method step b) two identifiers are assigned to the monitoring statuses of the at least one monitoring period (10) of the monitoring unit (5) of the first breathing apparatus (2), wherein preferably one identifier identifies the patient and one identifier identifies the at least one monitoring period (10).

4. The method according to any of the preceding claims, **characterized in that** the monitoring statuses of the at least one monitoring period (10) detected in method step a) are transmitted to a monitoring information system (4) together with the identifier assigned in method step b), wherein the monitoring statuses of the at least one monitoring period (10) detected in method step a) are stored at the monitoring information system (4) under the identifier assigned in method step b).

5. The method according to any of the preceding claims, **characterized in that** data for the identification of the monitoring statuses of the at least one monitoring period (10) of the monitoring unit (5) of the first breathing apparatus (2) are stored on a readable memory element (7).

6. The method according to claim 5, **characterized in that** the memory element (7) is provided in an accessory part, wherein the accessory part is preferably formed as a breathing tube (6).

7. The method according to claim 6, **characterized in that** in method step c) the accessory part is connected to the second breathing apparatus (3), wherein the data of the memory element (7) of the accessory part are read by a receiver unit (9) of the second breathing apparatus (3).

8. The method according to claims 3 and 7, **characterized in that** the monitoring statuses of the at least one monitoring period (10) of the first breathing apparatus (2) are requested from the monitoring information system (4) by the second breathing apparatus (3) under the identifier and transmitted to the second breathing apparatus (3).

9. The method according to any of the preceding claims, **characterized in that** the first breathing apparatus (2), the second breathing apparatus (3) and the monitoring information system (4) are interconnected by means of a data network.

10. The method according to any of the preceding claims, **characterized in that** a monitoring status comprises an activation and/or a deactivation of at least one monitoring variable.

11. The method according to claim 10, **characterized in that** the at least one monitoring variable comprises an Sp02 measurement, a CO2 measurement, an 02 measurement and/or a measurement of the concentration of an anesthetic.

12. The method according to claim 10, **characterized in that** in method step d) the activation and/or the deactivation of the at least one monitoring variable is taken into account.

13. The method according to any of the preceding claims, **characterized in that** in method step d) at least one monitoring variable is activated and that, preferably, the activation of the at least one monitoring variable takes place with a time delay.

## Revendications

1. Procédé pour la transmission d'états de surveillance entre des dispositifs respiratoires qui présente les étapes de procédé suivantes :
a) détection et enregistrement d'états de surveillance d'au moins une section de surveillance (10) d'une unité de surveillance (5) d'un premier dispositif respiratoire (2) et
b) assignation d'au moins une identification aux états de surveillance de la section de surveillance qui existe au moins (10) de l'unité de surveillance (5) du premier dispositif respiratoire,
**caractérisé par** une
c) transmission de l'identification qui existe au moins et des états de surveillance de la section de surveillance qui existe au moins (10) de l'unité de surveillance (5) du premier dispositif respiratoire (2) à un second dispositif respiratoire (3) et
d) réglage de l'unité de surveillance (5) du second dispositif respiratoire (3) conformément aux états de surveillance transmis de la section de surveillance qui existe au moins (10) de l'unité de surveillance (5) du premier dispositif respiratoire (2), les états de surveillance présents de l'unité de surveillance (5) du second dispositif respiratoire (3) étant pris en compte lors du réglage de l'unité de surveillance (5) du second dispositif respiratoire (3) de telle manière qu'un écart des états de surveillance présents du second dispositif respiratoire (3) par rapport aux états de surveillance de la section de surveillance qui existe au moins (10) du premier dispositif respiratoire (2) est signalisé par le second dispositif respiratoire (3).

2. Procédé selon la revendication 1, **caractérisé en ce que** les étapes a) à d) sont répétées pour les états de surveillances de chaque section de surveillance supplémentaire (10) de l'unité de surveillance (5) du premier dispositif respiratoire (2).

3. Procédé selon l'une des revendications précédentes, **caractérisé en ce que** dans l'étape de procédé b) deux identifications sont assignées aux états de surveillance de la première section de surveillance qui existe au moins (10) de l'unité de surveillance (5) du premier dispositif respiratoire (2), cependant que de préférence une identification caractérise le patient et une identification la section de surveillance qui existe au moins (10).

4. Procédé selon l'une des revendications précédentes, **caractérisé en ce que** les états de surveillancedétectés dans l'étape de procédé a) de la section de surveillance qui existe au moins (10) sont transmis ensemble avec l'identification assignée dans l'étape de procédé b) à un système d'information de surveillance (4), les états de surveillance détectés à l'étape de procédé a) de la section de surveillance qui existe au moins (10) étant déposés sur le système d'information de surveillance (4) sous l'identification assignée dans l'étape de procédé b).

5. Procédé selon l'une des revendications précédentes, **caractérisé en ce que** des données pour l'identification des états de surveillance de la section de surveillance qui existe au moins (10) de l'unité de surveillance (5) du premier dispositif respiratoire (2) sont déposées sur un élément de mémorisation qui peut être consulté (7).

6. Procédé selon la revendication 5, **caractérisé en ce que** l'élément de mémorisation (7) est prévu dans un accessoire, l'accessoire étant configuré de préférence comme un tuyau respiratoire (6).

7. Procédé selon la revendication 6, **caractérisé en ce que** dans l'étape de procédé c) l'accessoire est relié au second dispositif respiratoire (3), cependant que les données de l'élément de mémorisation (7) de l'accessoire sont lues par une unité réceptrice (9) du second dispositif respiratoire (3).

8. Procédé selon les revendications 3 et 7, **caractérisé en ce que** les états de surveillance de la section de surveillance qui existe au moins (10)du premier dispositif respiratoire (2) sont demandés par le second dispositif respiratoire (3) sous l'identification du système d'information de surveillance (4) et sont transmis au second dispositif respiratoire (3).

9. Procédé selon l'une des revendications précédentes, **caractérisé en ce que** lepremier dispositif respiratoire (2), le second dispositif respiratoire (3) et le système d'information de surveillance (4) sont reliés l'un à l'autre par un réseau de données.

10. Procédé selon l'une des revendications précédentes, **caractérisé en ce qu'**un état de surveillance comprend une activation et/ou une désactivation d'au moins une grandeur de surveillance.

11. Procédé selon la revendication 10, **caractérisé en ce que** la grandeur de surveillance qui existe au moins comprend une mesure de Sp02, une mesure de C02, une mesure d'02 et/ou une mesure de la concentration de la substance anesthésique.

12. Procédé selon la revendication 10, **caractérisé en ce que** dans l'étape de procédé d) l'activation et/ou la désactivation de la grandeur de surveillance qui existe au moins sont prises en compte.

13. Procédé selon l'une des revendications précédentes, **caractérisé en ce que** dans l'étape de procédé d) au moins une grandeur de surveillance est activée et de préférence l'activation de la grandeur de surveillance qui existe au moins se fait avec une temporisation.
